# EUROPEAN PATENT APPLICATION

(11) **EP 3 900 662 A1**
(43) Date of publication of application: **27.10.2021**
(21) Application number: 21169962.4
(22) Date of filing: 22.04.2021
(51) Int. Cl.: A61B 90/40

(54) **MEDICAL PROTECTIVE DEVICE**

(30) Priority: 22.04.2020 IT 202000008608
(71) Applicant: Casagrande Srl, 30125 Santa Lucia di Piave (TV) (IT)
(72) Inventor: Rigoni, Nicola, 36012 Asiago, Vicenza (IT); Rigoni, Simone, 31020 San Pietro di Feletto, Treviso (IT); Casagrande, Roberta, 31020 San Pietro di Feletto, Treviso (IT)
(74) Representative: Feira, Edoardo

(57) **Abstract**

The invention relates to a protective device (1000) for at least partially isolating the volume around the head of a patient lying in a substantially supine position, said protective device comprising: a surface (1100) having a substantially concave shape, wherein the surface (1100) has a first aperture (APE₁) of sufficient size to allow the insertion of the head of an adult person in an internal volume defined by the surface (1100), wherein the surface (1100) has one or more passages (1210), each of sufficient size to allow the insertion of a hand of an adult person.

## Description

### Field of the invention

The invention generally relates to a protective device of the medical type and, more specifically, to a protective device for at least partially isolating the volume around the head of a patient lying in a substantially supine position.

### Background

Following the recent pandemic caused by the SARS-CoV-2 virus, it became apparent that there was a need for protection against a highly infectious virus with transmission by aerosols and/or body fluids.

In particular, in the medical field, it is crucial to prevent the transmission from an infected patient to a healthcare worker. It is equally important to prevent a healthcare worker who may be infected without his knowledge from infecting an uninfected patient who is undergoing medical treatment for other reasons.

A particularly risky phase, both for the healthcare worker and the patient, is when the healthcare worker is working in an area close to the patient's face. This is the case, for example, with the intubation procedures, in the context of intensive medical care, but not only. Similar cases may be that of a dentist, an ophthalmologist, or any medical professional who needs to perform treatments that require proximity to the patient's face.

In particular, in these treatments, for example during the intubation phase or in the case of dental treatment, it is often impossible for the patient to wear a protective mask.

Furthermore, even with a mask on, contagion through aerosol and/or sweat, saliva, or other liquids emitted by the patient and/or healthcare worker is very high.

It is therefore necessary to provide a protective device that allows the healthcare worker to carry out the treatment in safety for himself and for the patient.

### Summary

The invention is based on the general concept that, instead of using a protective device worn by the patient, such as a mask, it is possible to use a larger sized device, which at least partially isolates the volume around the patient's face, so as to prevent the passage of liquids and/or aerosols between the patient and the healthcare worker. The invention is in particular defined by the independent claim.

In particular, a protective device for at least partially isolating the volume around the head of a patient lying in a substantially supine position may comprise: a surface having a substantially concave shape, where the surface may have a first aperture of sufficient size to allow the insertion of the head of an adult person in an internal volume defined by the surface, wherein the surface may have one or more passages, each of sufficient size to allow the insertion of a hand of an adult person.

In this way it is possible to use the surface to delimit the volume around the patient's head and thus contain the risk of contagion.

In some embodiments, the device may have at least one first axis of longitudinal extension intersecting the surface, wherein a first angle defined by the intersection between the surface and a first plane, perpendicular to the first axis of longitudinal extension, and having centre in the first axis of longitudinal extension, may be at least 180°, preferably at least 230°, even more preferably at least 270°.

In this manner, it is possible to delimit the volume around the patient's head as much as possible while still ensuring a sufficient aperture for the passage of the patient's head and/or shoulders.

In some embodiments, a first perimeter defined by the intersection between the surface and the first plane may increase as the distance of the first plane from a point of intersection of the first axis of longitudinal extension and the surface increases.

In this way it is possible to make the device with a dome-shape, i.e. with a section that increases downwards in the direction of the patient's head.

This allows greater stability of the device while ensuring effective volume delimitation around the patient's head and a limited size of the device.

In some embodiments, the device may also comprise a second aperture substantially parallel to the plane.

This makes it possible to advantageously position the device on a patient lying down, through the second aperture.

In some embodiments, the internal volume of the device may be comprised between 0.1 m³ and 0.4 m³. In some embodiments, the surface of the device may be comprised between 0.80 m² and 2.25 m².

These values allow a sufficient volume around the patient's head that is sufficient for containing the head itself and the movement of the hand(s) of the healthcare worker(s). In some embodiments, the surface can be made of a rigid, transparent material.

In this way it is possible for the health worker(s) to act in the best possible way on the patient without being exposed to risks.

In some embodiments, the one or more passages may be in a substantially opposite position to the first aperture.

Thanks to this configuration it is possible for the healthcare worker to position himself in the furthest, and therefore safest, area of the first aperture,

In some embodiments, each of the one or more passages may have an area greater than 100 cm² and/or less than 200 cm².

In this way, it is possible to allow the insertion of a hand and/or parts of the arm while ensuring a small size, reducing the risk of contagion.

In some embodiments, the surface may have one or more recesses for the at least partial insertion of the patient's shoulders.

In this way, it is possible to allow the partial insertion of the patient's shoulders, thus ensuring that the head is positioned deep in the device, reducing the risk of contagion. In some embodiments, the device may also comprise one or more anchoring elements configured to connect a flexible element covering at least partially the first aperture to the protective device.

This allows the risk of contagion to be further reduced by closing off the part of the first aperture not occupied by the patient's body with the flexible element.

In some embodiments, the surface may have one or more sides connected to each other by hinges.

This allows the device to be folded at least partially on itself when not in use.

### Brief description of the figures

Further characteristics and advantages of the invention will be more evident from the following description, set forth referring to the enclosed figures which show some nonlimiting embodiments thereof, where identical or corresponding parts of the device are identified by the same reference numbers. In the figures, any dashed lines represent parts that are not visible because they are covered by other parts, or shading to make the orientation of the surfaces clearer. In particular:
- Figure 1A schematically represents a perspective view of a protective device 1000;
- Figure 1B schematically represents a side view of the protective device of Figure 1A;
- Figure 1C schematically represents a side view of the protective device of Figure 1A;
- Figure 1D schematically represents a top view of the protective device of Figure 1A;
- Figure 1E schematically represents a side view of the protective device of Figure 1A;
- Figure 2 schematically represents a side view of a protective device 2000;
- Figure 3 schematically represents a side view of a protective device 3000;
- Figure 4 schematically represents a top view of a protective device 4000;
- Figure 5A schematically represents a perspective view of a protective device 5000;
- Figure 5B schematically represents a side view of the protective device of Figure 5A;
- Figure 5C schematically represents a top view of the protective device of Figure 5A;
- Figure 6 schematically represents a passage 6200;
- Figure 7 schematically represents a flexible segment 7210;
- Figure 8 schematically represents a perspective view of a protective device 8000;
- Figure 9 schematically represents a perspective view of a protective device 9000;
- Figure 10 schematically represents a perspective view of a protective device 10000;
- Figure 11A schematically represents a top view of a protective device 11000;
- Figure 11B schematically represents a side view of the protective device in Figure 11A.

### Detailed description of preferred implementation embodiments

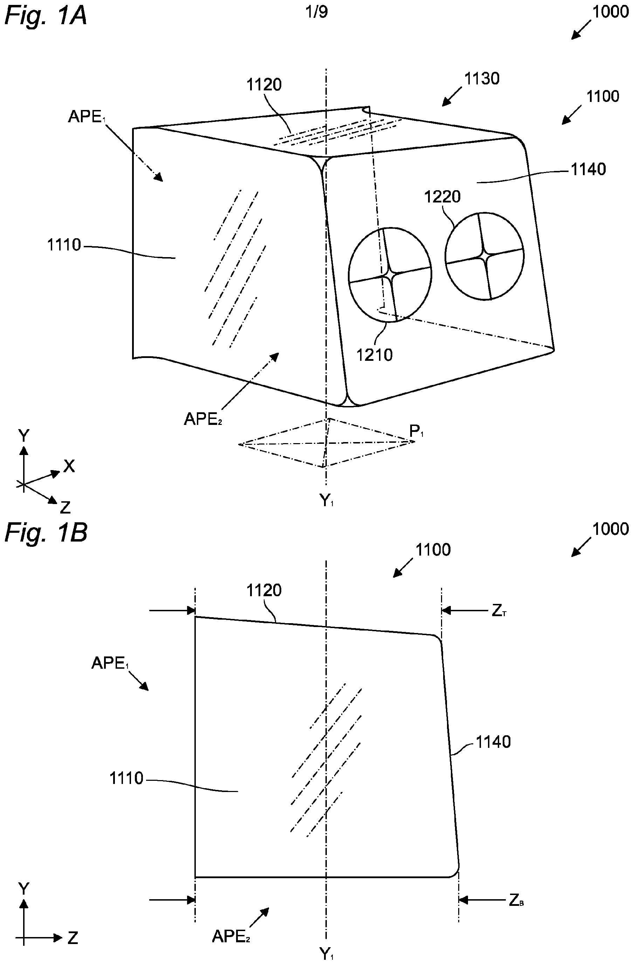

Figure 1A schematically represents a perspective view of a protective device 1000.

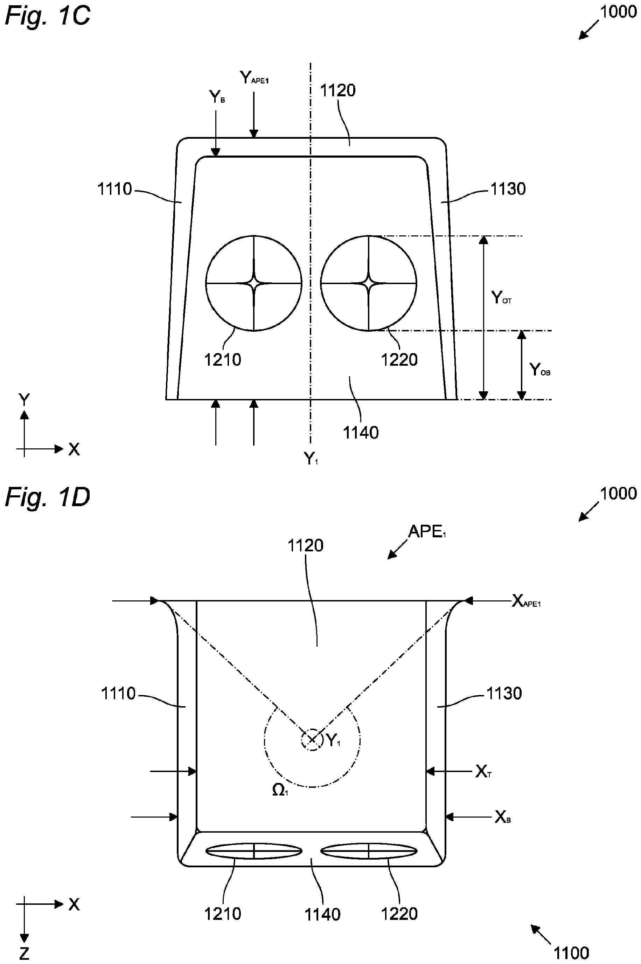

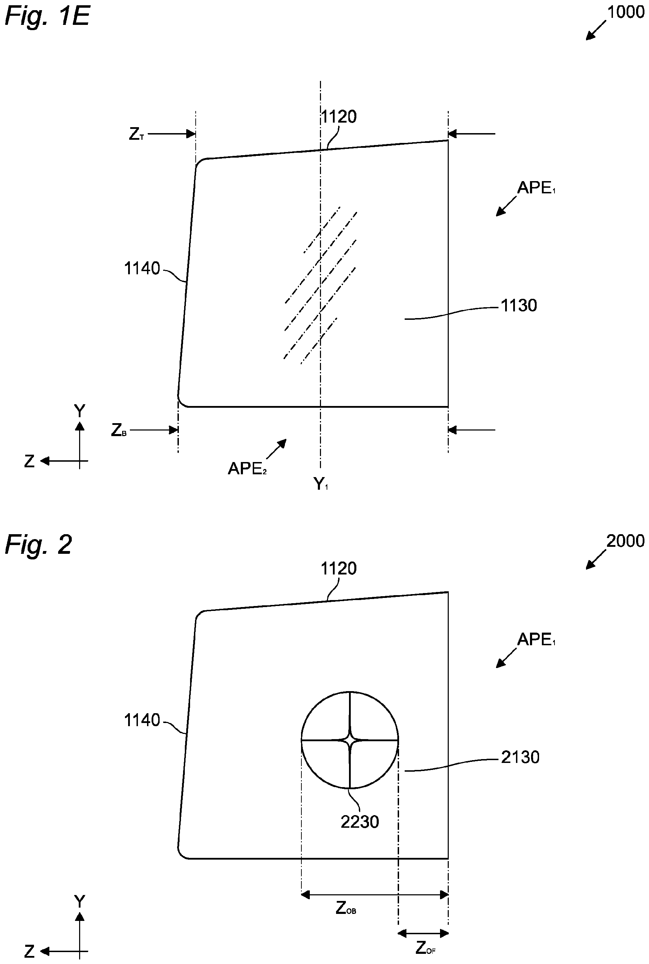

Figures 1B, 1C and 1E schematically represent several side views of the protective device 1000 of Figure 1A. Figure 1D schematically represents a top view of the protective device 1000 of Figure 1A. In the figures, the X, Y and Z axes represent Cartesian axes perpendicular to each other. References to "side" and "top" are made in relation to the position of the device 1000 as illustrated in the figures, which is also essentially the preferred position of use.

In the following, reference will also be made to the front, rear and lateral side of device 1000. Typically, the front side has an aperture that allows the patient to insert his head into the device, hereafter referred to as APE₁. The rear side is the one that is substantially parallel and opposite to the front side, with respect to the central portion of the device. The one or more lateral sides are those that connect the front to the rear side.

The protective device 1000 is generally configured to at least partially isolate the volume around the head of a patient lying in a substantially supine position. The head, not illustrated in the figures, is generally covered by the protective device 1000 so as to avoid risks of contagion through exchange of fluids and/or aerosols between the patient and the one or more medical professional(s).

In order to be able to operate in this manner, the protective device 1000 comprises a surface 1100 having a substantially concave shape. This concave shape generally allows the patient's head to be contained within the volume defined by it, so that the surface 1100 is positioned between the patient's head and the one or more medical professional(s). In particular, the surface 1100 may have a first aperture APE₁ of sufficient size to allow the insertion of the head of an adult person in an internal volume defined by the surface 1100.

In this manner, it is possible to allow the protective device 1000 to be positioned over the patient, with the neck or the chest substantially in proximity to the first aperture APE₁, or to move the patient, or to allow the patient to move himself, so as to position the head inside the protective device 1000, until the neck or the chest is substantially brought in proximity to the first aperture APE₁. In preferred implementation embodiments, it will be possible to position the head of the patient substantially close to the rear side of the device, for example the surface 1140, in the implementation embodiment illustrated in Figure 1.

In preferred implementation embodiments of the invention, the first aperture APE₁ may have a height Y_{APE1}, illustrated schematically for example in Figure 1C, preferably comprised between 35 cm and 75 cm, preferably 50 cm. In preferred implementation embodiments of the invention, the first aperture APE₁ may have a width X_{APE1}, illustrated schematically for example in Figure 1D, preferably comprised between 40 cm and 80 cm, preferably 55 cm.

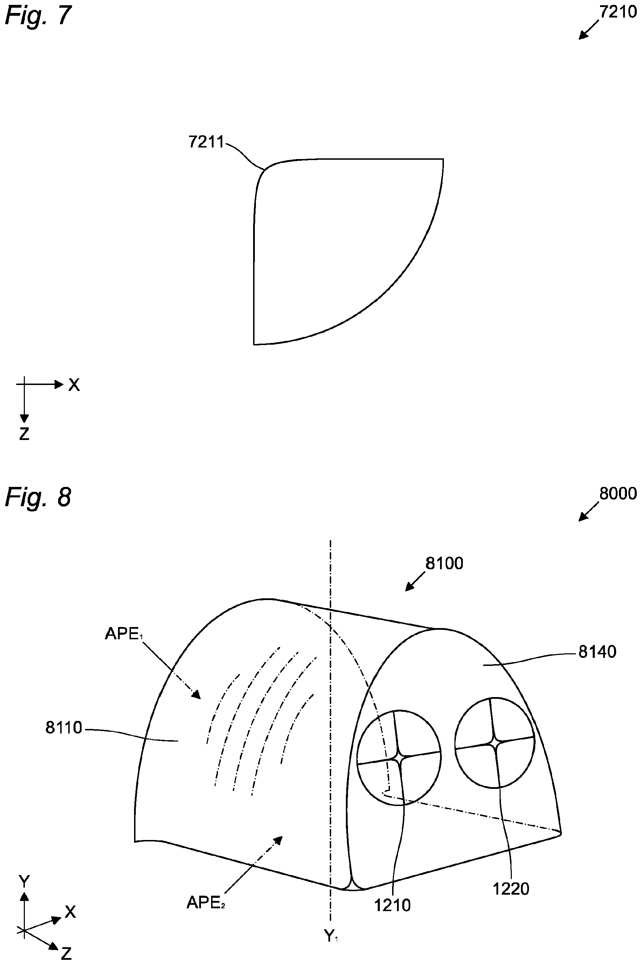

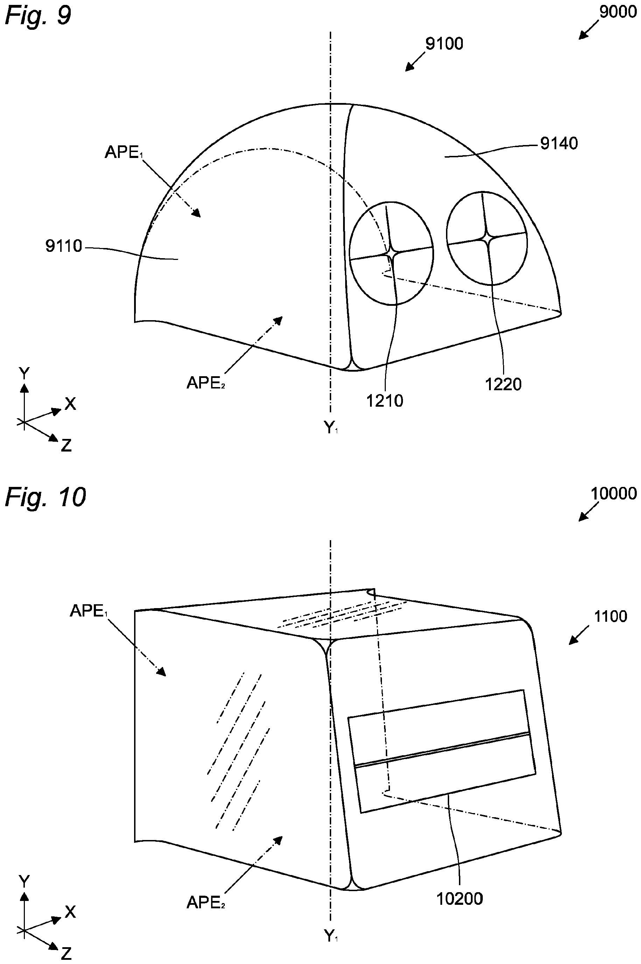

In the implementation embodiment illustrated in Figure 1, the first aperture APE₁ has a substantially rectangular, or trapezoidal, shape, it will however be clear that, in a more generic manner, any shape that allows at least the passage of the head of an adult may alternatively be operated, for example a substantially semicircular shape, as illustrated schematically in Figures 8 and 9. In preferred implementation embodiments, the shape and size of the aperture APE₁ may also be such as to allow the passage of the patient's shoulders.

The surface 1100 further has one or more passages 1210, 1220, each of sufficient size to allow the insertion of a hand of an adult person, in some implementation embodiments each of sufficient size to allow the insertion of the forearm of an adult person. In this way, it is possible for the one or more healthcare workers to insert one or more hands, possibly even part of the arm, within the volume defined by the surface 1100, so as to access the volume in which the patient's head is located, in order to carry out the necessary medical treatment.

In some implementation embodiments, the protective device 1000 may have a height, width and depth each comprised between 25 cm and 80 cm, preferably each comprised between 40 cm and 60 cm so as to allow a sufficient internal volume for the insertion of the patient's head, of the one or more hands of the healthcare worker and any medical devices to be provided. Advantageously, as far as the latter are concerned, it will also be possible to insert them from the one or more passages 1210, 1220. Alternatively or additionally, in particular in the case where such medical devices have sizes not compatible with the one or more passages 1210, 1220 it will be possible to insert them from the first aperture APE₁ and handle them once inserted in said volume.

In the implementation embodiment illustrated in Figure 1, the surface 1100 comprises substantially of four sides 1110, 1140, so as to form a substantially cube shape with the absence of at least one side, preferably two sides, wherein one absent side represents the first aperture APE₁ and the second absent side represents a second aperture APE₂, which is to be considered optional and will be described in more detail below.

It will be clear that the invention is not limited to a substantially cubic shape of the device 1000, like for example schematically illustrated by the alternative of Figure 8, where the protective device 8000 has a substantially barrel vault shape, with a curved surface 8110 having an arc shape and a substantially flat side 8140 having a function similar to the side 1140 of Figure 1. Again as an alternative, Figure 9 schematically illustrates a protective device 9000 having a shape substantially equal to that of a dome, of which the sides 9110 and 9140 are visible. Again alternatively, or in addition, the device may generally have a dome shape of other per se known types.

More specifically, in some implementation embodiments, the protective device may have at least a first axis of longitudinal extension Y₁ intersecting the surface 1100, 8100, 9100. In the figures, the first axis of longitudinal extension Y₁ is substantially parallel to the vertical axis Y and/or perpendicular to the plane XY on which at least the head of the patient is located. Additionally, or alternatively, in some implementation embodiments the first axis of longitudinal extension Y₁ may cross the device 1000 at a substantially central position of the device 1000 when viewed from above. Additionally, or alternatively, in some implementation embodiments the first axis of longitudinal extension Y₁ may be substantially perpendicular to the upper surface of the device, for example the surface 1120, and/or to the part of the upper surface of the device intersected by the first axis of longitudinal extension Y₁.

The intersection between the surface 1100, 8100, 9100 and a first plane P₁, perpendicular to the first axis of longitudinal extension Y₁, defines a perimeter of the surface 1100, 8100, 9100, which may be constant at different heights Y or may be variable, for example due to variations in the size of the first aperture APE₁ and/or the shape of the protective device. This perimeter defines a first angle Ω₁, having centre in the first axis of longitudinal extension Y₁, which is preferably of at least 180°, preferably at least 230°, even more preferably at least 270°. The remaining angle, with respect to the full 360° angle, is therefore the angular extension of the first aperture APE₁. This angular aperture advantageously allows the device 1000 to ensure an easy insertion and removal of the patient's head, while providing a wide angular direction of protection, given by the surface 1100, so that the one or more healthcare workers can safely position themselves on different sides of the patient's head. In some implementation embodiments, the angular values given above are valid for at least 1/4 of the overall height of the protective device, preferably for at least 1/2 of the overall height of the protective device.

In some implementation embodiments, the perimeter described above, defined by the intersection between the surface 1100, 8100, 9100 and the first plane P₁, can advantageously increase as the distance of the first plane P₁ from a point of intersection of the first axis of longitudinal extension Y₁ and the surface 1100, 8100, 9100 increases. In addition or alternatively, the maximum sizes of said perimeter on the plane P₁ may increase as the distance of the first plane P₁ from a point of intersection of the first axis of longitudinal extension Y₁ and the surface 1100, 8100, 9100 increases.

Thanks to this configuration, the protective device 1000 generally widens in the plane XY, going downwards. This is advantageous as it allows for a better stability of the protective device against accidental tipping over, particularly in the presence of the second aperture APE₂ in the base of the protective device 1000. This situation is schematically illustrated, for example, in Figures 1B and 1E, wherein the depth Z_{T} at the top part of the device 1000, is less than the depth Z_{B} at the bottom part of the device 1000, and/or in Figure 1D, wherein the width X_{T} at the top part of the device 1000, is greater than the width X_{B} at the bottom part of the device 1000. In some implementation embodiments of the invention, the side 1140 may have an inclination with respect to the vertical direction Y comprised between 5 and 20 degrees, preferably about 10 degrees. In some embodiments of the invention, the depth Z_{B} at the bottom part of the device 1000 may be comprised between 30 cm and 50 cm.

In some implementation embodiments, the protective device 1000 may further comprise a second aperture APE₂ substantially parallel to the first plane P₁. This implementation is, for example, the one illustrated in Figures 1A-1E. The presence of the second aperture APE₂ advantageously allows the device 1000 to be positioned above the head of a patient, without moving the patient. However, it will be clear that, in implementation embodiments not illustrated, a further side of the surface 1100 may instead be present in place of the second aperture APE₂. The latter implementation offers, for example, a better structural rigidity to the protective device, as well as preventing it from accidentally tipping over as it is advantageously held in place by the weight of the patient's head.

In some implementation embodiments, the internal volume is comprised between 0.1 m³ and 0.4 m³, more preferably between 0.1 m³ and 0.2 m³. The internal volume shall mean the volume defined by the surface 1100 and delimited by a plane closing the first aperture APE₁ and, if any, by a plane closing the second aperture APE₂. This volume allows enough space for the insertion of the patient's head into the protective device, as well as the one or more of the healthcare worker's hands and any medical devices. At the same time, this volume allows the patient, when conscious, not to feel trapped in a too narrow space. Furthermore, these sizes allow the protective device to be positioned on a standard-sized hospital bed.

In some implementation embodiments, the surface 1100, 8100, 9100 has an area comprised between 0.80 m² and 2.25m², more preferably between 0.80 m² and 1.5m². These sizes allow obtaining the same advantages associated with the volume sizes described above to be provided.

In some implementation embodiments, the surface 1100, 8100, 9100 is made of a rigid, transparent, preferably thermoplastic material, so that the protective device can be obtained by plastic moulding, in a cost-effective and efficient manner, and at the same time allow the medical professional to have a complete view of the patient's head.

In some implementation embodiments, the one or more passages 1210, 1220 are in substantially opposite positions with respect to the first aperture APE₁ for example as illustrated in the case of the protective device 1000. Opposite direction means opposite to the first axis of longitudinal extension Y₁. This allows the medical professional to position himself in the furthest, and therefore most protected, position from the first aperture APE₁. Moreover, this position is the natural one in the case of certain procedures, such as for example intubation.

The number and shape of the one or more passages 1210, 1220 is not limited to that illustrated in Figure 1A, where there are two substantially circular passages 1210, 1220 on the side 1140. By way of example, it will be possible to replace these two passages 1210, 1220, with a single passage 10200, like for example illustrated in Figure 10.

In some implementation embodiments, the sizes of the passage 10200 may be equal to the sum of the sizes of two passages 1210, 1220, positioned side by side. In some implementation embodiments it will also be possible for the passage to be implemented as a sleeve crossing the rear plane of the device, for example the plane 1140, with an outward extension from the device of at least 1cm, so as to further reduce the risk of contagion.

Again in addition, or as an alternative, it will be possible to provide for one or more passages 1210, 1220 also on different sides of the device, like for example illustrated by the passage 2230 illustrated in Figure 2, on the side 2130. The passage 2230 has a position substantially perpendicular to that of the one or more passages 1210, 1220, so as to advantageously allow the patient's head to be reached from a different angle. It will be clear that passages may also be present on other sides of the device. For example, with reference to Figure 1A, in addition to the already mentioned sides 1140 and 1130 it will be possible to implement passages on one or more of the sides 1110, 1120. In general, one or more passages may be implemented on one or more lateral sides, for example 1110, 1130, and rear side, for example 1140, of the protective device.

In addition, the presence of several passages allows several healthcare workers to act simultaneously, which may be for example necessary in the case of an intubation, during which the operator performing the actual intubation is preferably positioned at the one or more passages 1210, 1220 while a second and/or third operator assists/assist in a lateral position, for example from the sides 1110, 1130, at the one or more passages 2230.

In preferred embodiments of the invention, two passages, or a passage of sufficient size for the insertion of two hands, will be made, preferably on the rear side, for example on the side 1140. In addition, in some implementation embodiments, a passage will be made on at least one lateral side, for example the side 1110. In some implementation embodiments, one passage will be made on each lateral side, e.g. the sides 1110, 1130, thus resulting in two lateral passages.

In some implementation embodiments, each of the one or more passages 1210, 1220, 2230, 10200 preferably has an area greater than 100 cm² and/or preferably less than 200 cm², particularly if intended for one-handed use. This size advantageously allows ensuring an easy insertion of the hand, possibly also parts of the arm, of an adult operator, and having sufficient freedom of movement for the operator. At the same time, the limitation of the area makes it possible to avoid accidental contagion through the one or more passages 1210, 1220, 2230, 10200.

In some implementation embodiments, as illustrated in Figure 1C, the one or more passages 1210, 1220, 2230, 10200 may be open starting from a height Y_{OB} preferably comprised between 15 cm and 25 cm. Furthermore, in some implementation embodiments, as illustrated in Figure 1C, the one or more passages 1210, 1220, 2230, 10200 may be open up to a height Y_{OT} preferably comprised between 30 cm and 40 cm. The height is preferably measured from the lowest point of the device 1000 and/or, in the implementation embodiments that comprise it, from the second aperture APE₂. These values advantageously allow the healthcare worker to reach comfortably the patient's face, while maintaining a comfortable operating arm angle for the healthcare worker, and at the same time keeping a size of the passages 1210, 1220, 2230, 10200 small, so as to reduce the risk of contagion.

In addition, or alternatively, the one or more passages 2230 may be open starting from a depth Z_{OF} preferably comprised between 5 cm and 15 cm. Furthermore, in some implementation embodiments, as illustrated in Figure 1C, the one or more passages 2230 may be open up to a depth Z_{OB} preferably comprised between 20 cm and 30 cm. The depth is preferably measured from the first aperture APE₁. These values advantageously allow the healthcare worker to operate in the area of the patient's face while maintaining a comfortable arm operating angle, as described above. Furthermore, these sizes advantageously allow inserting at least one hand in one of the passages made on the rear side 1140, for example the passage 1220, and at the same time at least one hand in one of the passages made on a lateral side 1110, 1130, for example the passage 2230, allowing both hands to operate in the volume defined by the device.

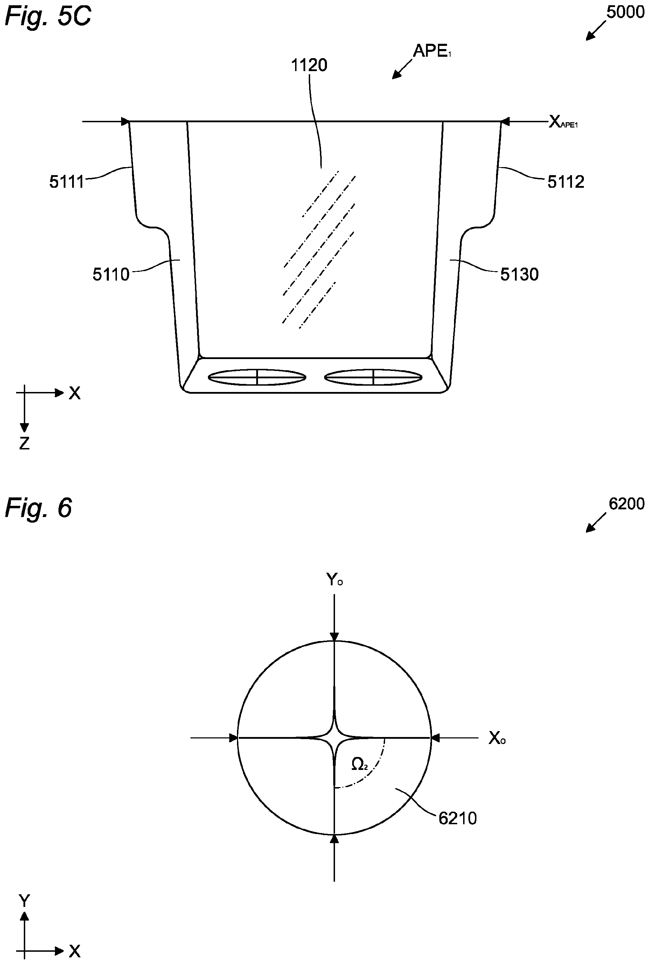

In some implementation embodiments, in order to reduce the risk of accidental contagion through the one or more passages 1210, 1220, 2230, 10200 it will be possible to provide for the presence of one or more flexible segments 6210, as illustrated for example in Figure 6, for one or more passages. In particular, the one or more flexible segments may be made of flexible material and installed on the one or more passages so as to require the greater part of the passage, preferably the whole of the passage, in the absence of other forces acting on them. However, the flexibility of the material of the flexible segments 6210 allows them to be folded so that it is possible for the healthcare worker to insert his hand inside the passage.

In the particular case of a passage 6200 having a substantially circular shape, it will be possible to implement a plurality N of flexible segments 6210 having a substantially isosceles triangle shape, each having an angle Ω₂ directed towards the centre of the passage 6200 having a value of about 360°/N, preferably of 350°/N, so as to maintain a sufficient closure by avoiding direct contact between the various flexible segments. In preferred implementation embodiments, N is advantageously comprised between 4 and 20.

In some implementation embodiments, as illustrated in Figure 7, it will be possible to provide for a rounded corner of the flexible segment 7210, preferably the corner towards the centre of the passage, so as to prevent the tip of the flexible segment 7210 from being scratched and/or piercing any gloves and/or the operator's skin when the operator's hand is withdrawn, thereby creating a possible nuisance as well as a risk of contagion. In preferred implementation embodiments the rounded corner can have a radius of curvature greater than 0.5cm, preferably greater than 1cm, to ensure comfort of use without creating a too large aperture in the passage. Alternatively, or in addition one or more of the flexible segments 6210, preferably each of the flexible segments 6210, may be made of flexible material, such as for example silicone material, latex, rubber, nitrile, neoprene, thereby reducing the risk of tearing the healthcare worker's gloves.

In some implementation embodiments, the protective device may further comprise one or more recesses 3111, 4111, 4112, 5111, 5112 for the at least partial insertion of the patient's shoulders. Generally the recesses allow the shoulders of the patient to be at least partially inserted into the protective device, thus ensuring that the head of the patient is not too close to the first aperture APE₁ thus guaranteeing greater safety as well as easier action by the healthcare worker, as the head of the patient is brought closer to the one or more of the rear passages 1210, 1220, 10200. At the same time, instead of making the whole protective device wider, so as to have a sufficient width to insert the patient's shoulders, with the presence of the recesses 3111, 4111, 4112, 5111, 5112 it will be possible to obtain a more compact, light and economical structure for the protective device, in addition to making the action of the healthcare worker easier, since the patient's head is brought closer to the one or more lateral passages 2230.

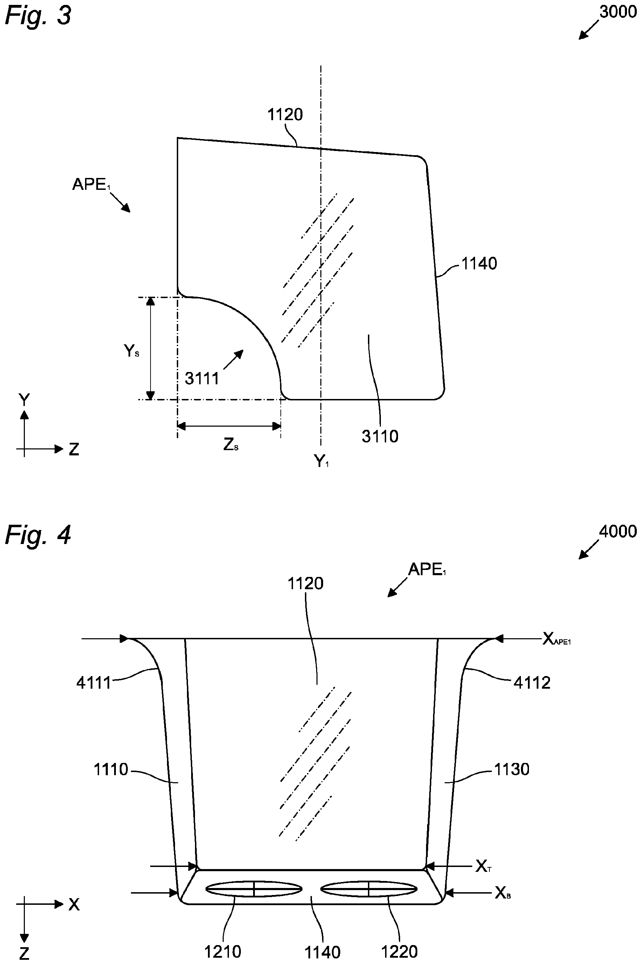

Figure 3 illustrates a first implementation embodiment of the recesses, in the form of recesses 3111. The recess 3111 is formed by a recess in the surface 1100 near the corner of the device between the first aperture APE₁ and the second aperture APE₂. The recess 3111 has a shape that generally allows at least part of the shoulders to be inserted into the surface 1100 and, in particular, into the sides 1110 and 1130, renumbered in Figure 3110. In some implementation embodiments the recess 3111 may have the shape of an arc of a circle or be polygonal in shape, more generally a concave shape in the direction of the angle of the protective device between the first aperture APE₁ and the second aperture APE₂. In some implementation embodiments the recess 3111 may have a height Y_{S} preferably comprised between 8 cm and 14 cm. In some implementation embodiments the recess 3111 may have a depth Zs preferably comprised between 8 cm and 14 cm.

Figure 4 illustrates an alternative implementation embodiment of the recesses, in the form of recesses 4111, 4112. The recesses 4111, 4112 are formed by increasing the width X_{APE1} of the protective device near the first aperture APE₁, with respect to the width X_{B}, X_{T} respectively in the bottom and top side of the protective device in the rear side, opposite the first aperture APE₁. Thanks to the widening of the front portion of the protective device, the one near the first aperture APE₁, it is possible to have more space in this area for the at least partial insertion of the shoulders. The difference between X_{APE1} and X_{B} and/or X_{T} can be at least 5 cm, preferably at least 10 cm. The widening can be made in the first 10 cm of the depth of the protective device, from the first aperture APE₁ in the direction of the rear side of the device.

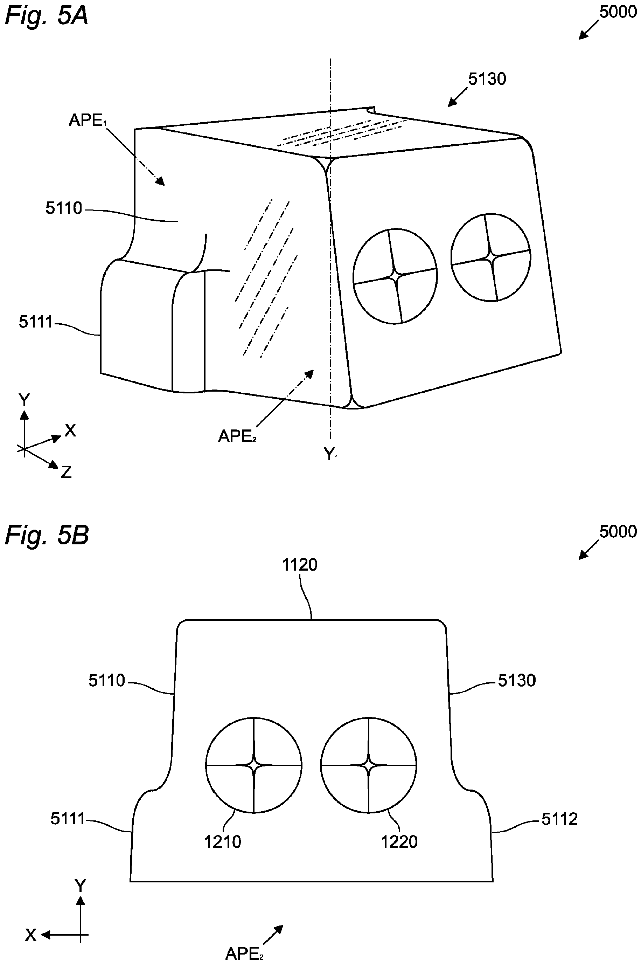

Figures 5A-5C schematically illustrate an alternative similar to the one in Figure 4, where the recesses 5111, 5112, functionally corresponding to the recesses 4111, 4112, have a more pronounced widening in the figures, so as to make them more obvious. Furthermore, in the implementation embodiment of Figures 5A-5C, the recesses 5111, 5112 have a height less than the height of the device, and preferably greater than 10cm and/or less than 25cm. In this way it is possible to maintain a compact structure for the safety device, while ensuring sufficient space for various shoulder sizes.

In some implementation embodiments, in particular those where the surface 1100, 8100, 9100 of the safety device has two or more sides forming an angle between them, such as for example the side 1110 with the side 1120, or for example the side 8110 with the side 8140, it will be possible to form the internal part of the surface at the point joining the two sides forming the angle so as to have an internal radius of curvature of at least 2 cm, preferably at least 4 cm. This solution advantageously avoids the formation of internal corners that could be difficult to clean and/or disinfect. Conversely, by forming the internal corners of the safety device with the indicated radii of curvature, it is possible to ensure an internal surface that is easy and quick to clean.

In some implementation embodiments, the protective device may further comprise one or more anchoring elements 11300 configured to connect a flexible element covering at least partially the first aperture APE₁ to the protective device. This generally allows obtaining a better closure of the first aperture APE₁. In addition, thanks to the flexibility of the flexible element, this closure can allow the movement of the patient through the first aperture APE₁ as well as adapting to the shape of the patient's neck and/or chest. In some implementation embodiments, the flexible element, not shown in the figures, may be, for example, a textile and/or plastic element, preferably washable and/or disposable.

The anchoring elements can be mechanical, for example elastic bands, buttons, clamps, and more generally any element that allows the textile element to be connected to the protective device. In addition or alternatively, the anchoring elements may be magnetic, for example magnets and/or ferromagnets, and the flexible element may also comprise magnetic and/or ferromagnetic materials. In addition or as an alternative, the anchoring elements can be implemented using Velcro. In addition or as an alternative, the anchoring elements can be implemented in the form of hooks on the external surface 1110, which are inserted into respective holes of the flexible element. In addition or alternatively, the anchoring elements can be implemented in the form of holes, passing through or not, in the external surface 1110, into which the respective hooks of the flexible element can be inserted.

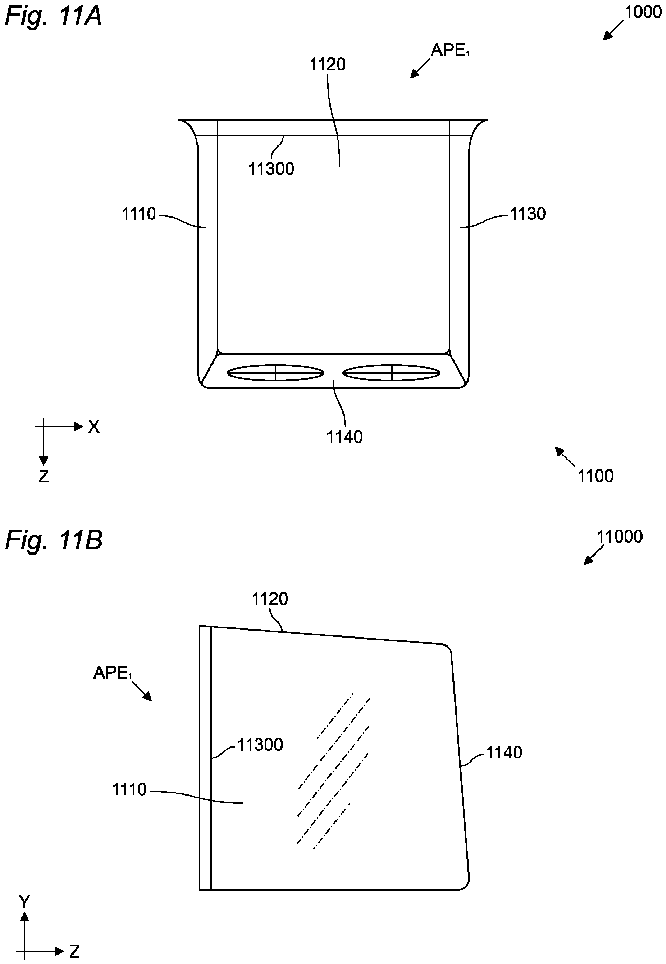

A possible implementation embodiment of the anchoring elements 11300 and/or a possible arrangement of the previously described anchoring elements is visible in Figure 11A and 11B. In this implementation embodiment, the anchoring elements 11300 are implemented by a recess and/or a protrusion in the protective device material adjacent to the first aperture APE₁. Adjacent, in some implementation embodiments, may mean that the anchoring elements are at a distance from the first aperture APE₁ of less than 10 cm, preferably less than 5 cm. Said recess and/or protrusion enables, in conjunction with elastic means which interact mechanically with the flexible element and said recess and/or protrusion, to connect and disconnect the flexible element from the protective device. However, it will be clear that any of the previously described anchoring elements, or a combination thereof, may be implemented instead of the aforesaid recess and/or protrusion. It will also be clear that, in some implementation embodiments, it will be possible to position the anchoring elements 11300 even on part of the perimeter of the first aperture APE₁.

In some implementation embodiments, the surface of the protective device may have one or more sides, e.g. the sides 1110-1140 for the surface 1100, the sides 8110, 8140 for the surface 8100, the sides 9110, 9140 for the surface 9100 connected to each other by hinges. This configuration advantageously allows the protective device to be closed on itself, thus reducing the overall storage overall footprints thereof. Alternatively, or additionally, the convex shape of the protective device may be configured so as to allow the at least partial insertion, preferably of at least the upper half, of a protective device within the volume of a second protective device, in other words by stacking the protective devices on top of each other. This solution, advantageously applicable to the convex structures illustrated in the figures, makes it possible to reduce the shipping overall footprints of a plurality of protective devices shipped in a single package.

The invention has been described with reference to various characteristics, possibly illustrated as belonging to a single implementation embodiment. However, it will be clear that for implementing the invention it will not be necessary to implement all the characteristics of a single implementation embodiment described and any combination of characteristics will form the basis of an implementation embodiment according to the invention. It will also be clear that features of different implementation embodiments can be combined with each other resulting in new implementation embodiments of the invention. More generally, the invention is understood as defined by the claims which follow, to which one or more of the characteristics defined above will be combinable, independently of other characteristics described in the same implementation embodiment.

### REFERENCE NUMBERS

1000: protective device
1100: surface
1110-1140: side
1210-1220: passage
APE₁, APE₂: aperture
P₁: plane
X_{B}, X_{T}, X_{APE}: width
Y_{B}, Y_{APE}, Y_{OB}, Y_{OT}: height
Y₁: axis of longitudinal extension
Ω₁: angle
Z_{B}, Z_{T}: depth
2000: protective device
2130: side
2230: passage
Z_{OB}, Z_{OF}: depth
3000: protective device
3110: side
3111: shoulder recess
Y_{S}: shoulder aperture height
Z_{S}: shoulder aperture depth
4000: protective device
4111, 4112: shoulder recess
X_{APE}: aperture width
5000: protective device
5111, 5112: shoulder recess
6200: passage
6210: flexible segment
X_{O}: aperture width
Y_{O}: aperture width
7210: flexible segment
7211: rounded end
8000: protective device
8100: surface
8110, 8140: side
9000: protective device
9100: surface
9110, 9140: side
10000: protective device 10200: passage
11000: protective device
11300: anchoring element

## Claims

1. Protective device (1000, 2000, 3000, 4000, 5000, 8000, 9000, 10000, 11000) for at least partially isolating the volume around the head of a patient lying in a substantially supine position, said protective device comprising:
a surface (1100, 8100, 9100) having a substantially concave shape,
wherein the surface (1100, 8100, 9100) has a first aperture (APE₁) of sufficient size to allow the insertion of the head of an adult person in an internal volume defined by the surface (1100, 8100, 9100),
wherein the surface (1100, 8100, 9100) has one or more passages (1210, 1220, 2230, 10200), each of sufficient size to allow the insertion of a hand of an adult person.

2. The protective device (1000, 2000, 3000, 4000, 5000, 8000, 9000, 10000, 11000) according to claim 1,
wherein the device has at least one first axis of longitudinal extension (Yi) intersecting the surface (1100, 8100, 9100),
wherein a first angle (Ω₁) defined by the intersection between the surface (1100, 8100, 9100) and a first plane (Pi), perpendicular to the first axis of longitudinal extension (Yi), and having centre in the first axis of longitudinal extension (Yi), is at least 180°, preferably at least 230°, even more preferably at least 270°.

3. The protective device (1000, 2000, 3000, 4000, 5000, 8000, 9000, 10000, 11000) according to claim 2,
wherein a first perimeter defined by the intersection between the surface (1100, 8100, 9100) and the first plane (Pi), increases as the distance of the first plane (Pi) from a point of intersection of the first axis of longitudinal extension (Yi) and the surface (1100, 8100, 9100) increase.

4. The protective device (1000, 2000, 3000, 4000, 5000, 8000, 9000, 10000, 11000) according to claim 2 or 3,
further comprising a second aperture (APE₂) substantially parallel to the plane (P1).

5. The protective device (1000, 2000, 3000, 4000, 5000, 8000, 9000, 10000, 11000) according to any one of the preceding claims,
wherein the internal volume is comprised between 0.1 m³ and 0.4 m³.

6. The protective device (1000, 2000, 3000, 4000, 5000, 8000, 9000, 10000, 11000) according to any one of the preceding claims,
wherein the surface (1100, 8100, 9100) is comprised between 0.80 m² and 2.25 m².

7. The protective device (1000, 2000, 3000, 4000, 5000, 8000, 9000, 10000, 11000) according to any one of the preceding claims,
wherein the surface (1100, 8100, 9100) is made of rigid, transparent material.

8. The protective device (1000, 2000, 3000, 4000, 5000, 8000, 9000, 10000, 11000) according to any one of the preceding claims,
wherein the one or more passages (1210, 1220, 2230, 10200) are in a substantially opposite position to the first aperture (APE₁).

9. The protective device (1000, 2000, 3000, 4000, 5000, 8000, 9000, 10000, 11000) according to any one of the preceding claims,
wherein each of the one or more passages (1210, 1220, 2230, 10200) has an area greater than 100 cm² and/or less than 200 cm².

10. The protective device (3000, 4000, 5000) according to any one of the preceding claims,
wherein the surface (1100, 8100, 9100) has one or more recesses (3111, 4111, 4112, 5111, 5112) for the at least partial insertion of the patient's shoulders.

11. The protective device (11000) according to any one of the preceding claims, further comprising one or more anchoring elements (11300) configured to connect a flexible element covering at least partially the first aperture (APE₁) to the protective structure.

12. The protective device (1000, 2000, 3000, 4000, 5000, 8000, 9000, 10000, 11000) according to any one of the preceding claims,
wherein the surface (1100, 8100, 9100) has one or more sides (1110-1140) connected to each other by hinges.
